# EUROPEAN PATENT APPLICATION

(11) **EP 1 340 745 A1**
(43) Date of publication of application: **03.09.2003**
(21) Application number: 02003241.3
(22) Date of filing: 20.02.2002
(51) Int. Cl.: C07D 209/36, A61K 31/404, A61P 35/00

(54) **Indirubin derivatives and their use for the treatment of cancer**

(71) Applicant: Eisenbrand, Gerhard, Prof. Dr., 69120 Heidelberg (DE)
(72) Inventor: Eisenbrand, Gerhard, Prof. Dr., 69120 Heidelberg (DE); Hippe, Frankie, 8221 Mamer (LU)
(74) Representative: Müller-Boré & Partner Patentanwälte

(57) **Abstract**

The present invention relates to novel indirubin derivatives having the following general formula 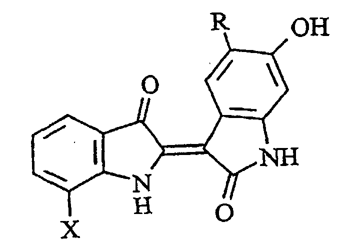 wherein the group R represents a straight-chain or branched-chain alkyl group or a straight-chain or branched-chain alkoxy group each having 1 to 18 carbon atoms, and X is H or OH, which can be used for the manufacture of a medicament for the treatment of solid cancers and leukemia and metastases therof.

## Description

The present invention relates to novel indirubin derivatives which can be used for the manufacture of a medicament for the treatment of solid cancers and leukemia and metastases thereof.

Indigoid bisindoles comprise a spectrum of natural dyestuffs. Many of these can be obtained from plants. Accordingly, indirubin, indigo and isoindigo which are all indigoid bisindoles derivatives, are natural products which can be obtained from different plants: namely, Baphicacanthus cusia (Acanthaceae), Indigofera suffruticosa (Fabaceae), Isatis indigotica (Brassicaceae) and others. Indican, a glycoside which is found in plants, gives glucose and 3-hydroxyindole due to acidic or enzymatic hydrolysis. 3-Hydroxyindole is converted by air-oxidation into indigo and its isomers. Indigo naturalis (Chinese: Quing Dai) is the natural blue dye obtained from plant material, e.g. Isatis indigotica (Brassicaceae). Indirubin, an isomer of indigo, can be found in Indigo naturalis as a by-product (Falbe J. & Regitz M., Römpp Chemie Lexikon (1992), 9. Aufl., Stuttgart, Georg Thieme Verlag). It occurs also in Isatis tinctoria in an amount of up to 5% which is indigenous to Central Europe (Gelius R., Z. Chem., 20, (1980), 340-341). Derivatives of indirubin are known for a long time as dyes of low persistence.

Indigo naturalis is reported to be used in traditional Chinese medicine as a haemostatic, antipyretic, antiinflammatory and sedative agent in the treatment of bacterial and viral infections. Antileukemic effects of Indigo naturalis have also been reported, with indirubin being the effective principle (Ji X. et al., Acta Pharm. Sin., 16, (1981), 146-148; Gan W. J. et al., J. Hematol., 6, (1985), 611-613). In spite of its antileukaemic activity, however, indirubin dissolves only poorly in water and is therefore not readily resorbed. Recently, the antileukemic activity of some better soluble indirubin derivatives has been reported (Ch. Li et a., Bull. Chem.

Soc. Jpn. **69**, 1621-1627,(1996)). For indigoid bisindoles, in order to be used in the treatment of human solid tumors and leukemia one of the major topics is to provide compounds with improved bioavailability.

Thus, the technical problem underlying the present invention is to provide new active substances which can be used in the treatment of human solid tumors and leukemia and metastases thereof. Furthermore, the absorbability of said substances should be improved in order to improve their bioavailability and their *in vivo* antitumor activity.

The solution to the above technical problem is achieved by the embodiments characterized in the claims.

In particular, the present invention relates to cell membrane penetrating indirubin derivatives having the following general formula 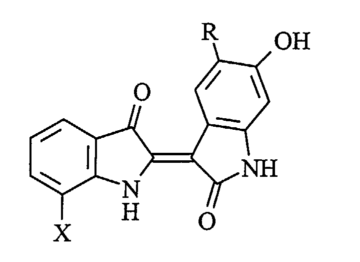 wherein the group R represents a straight-chain or branched-chain alkyl group or a straight-chain or branched-chain alkoxy group each having 1 to 18 carbon atoms, preferably 1 to 4 carbon atoms, and X is H or OH. Particularly, the group R stands for methyl, ethyl, n-propyl, n-butyl, methoxy, ethoxy, propoxy and butoxy.

Preferred compounds of the indirubin derivatives of the present invention are 6-hydroxy-5-methylindirubin and 6,7'-dihydroxy-5-methylindirubin.

The antitumor active compounds according to the present invention can be used for the manufacture of a medicament for the treatment of human solid tumors and leukemia and metastases thereof. The term "human solid tumors" according to the present invention preferably includes carcinomas, melanomas, adenomas, sarcomas, lymphomas, neuroblastomas, teratomas and astrocytomas. Specific examples are mammary carcinomas, lung carcinomas, stomach and liver carcinomas, colon carcinomas, bladder carcinomas, ovarian carcinomas, pancreatic carcinomas, renal carcinomas, prostatic carcinomas, bronchial carcinomas, laryngeal carcinomas and the like.

In the pharmaceutical formulations according to the present invention, the indirubin derivatives can also be employed in the form of their physiologically acceptable salts. The above identified indirubin derivatives of the present invention can be formulated into pharmaceutical compositions which contain optionally a pharmaceutically acceptable carrier and/or diluent. Said pharmaceutical compositions can be applied e.g. orally, topically, intravenously, intraperitoneally, subcutaneously and rectally in pharmaceutically effective amounts.

One general problem in the field of pharmacology is the formulation of pharmaceutically active substances in pharmaceutical compositions which can be applied to a human body. Since most physiological fluids are water-based, the pharmaceutically active substances should be soluble in water and/or a water miscible solvent wherein the latter of course has to be physiologically acceptable in small concentrations, such as ethanol. Furthermore, pharmaceutically active substances to be applied orally have to be absorbed into the human body and have to permeate the gastrointestinal mucous membrane to be taken up into the portal blood circulation to reach the liver. By the same token, compounds applied parenterally, e.g. intravenously, need to cross several membranes in order to reach and to penetrate the target cells of the tumor.

Cellular membranes are composed of lipid bilayers, i.e. composed of a rather nonpolar medium. Therefore, substitution with very polar groups on the one hand improves the water solubility of a compound but on the other hand hinders or even prohibits the absorption of antitumor active substances into a tumor cell. Thus, antitumor active substances which show good inhibitory activities under certain *in vitro* conditions towards proliferation related targets such as growth factor receptors, kinases and other signalling enzymes have to be rejected because of not showing any activity when tested using intact cells or organisms.

Therefore, the indirubin derivatives according to the present invention are cell membrane penetrating indirubines. According to the present invention the term "cell membrane penetrating" means the ability of the indirubin derivatives to be taken up by the tumor cell through the cellular membrane.

The chemical incorporation of an alkyl or alkoxy substituent into the 5-position of indirubin brings about a drastic change in biotransformation behaviour and makes 5-alkyl-substituted or 5-alkoxy-substituted inrubin derivatives easily accessible to oxidative metabolism. It has been found that this oxidative metabolism is mediated by cytochrom P450 dependent liver monooxygenases. Experiments in liver microsome preparations from cows, pigs and rats and also from a spectrum of human liver samples have shown that for example 5-methylindirubin is rapidly metabolized, yielding ring-hydroxylated indirubin derivatives as major metabolites besides small amounts of further metabolites.

5-Methylindirubin has been proven to be highly effective on human solid tumors implanted into nude mice without any perceptible toxicity. To the contrary, the parent compound indirubin is considerably less effective, but already remarkably toxic as evidenced by considerable body weight loss at a comparable dosage; cf. PCT/EP00/03210. It has now surprisingly been found that the incorporation of, for example, a methyl group or a methoxy group into the 5-position of indirubin renders the molecule easily metabolizable. This is in contrast to the unsubstituted parent compound indirubin which is not metabolized to a detectable extent under the same conditions. In particular, two main metabolites have been isolated and structurally determined, namely 6-hydroxy-5-methylindirubin and 6,7'-dihydroxy-5-methylindirubin. Both metabolites are at least as effective in inhibiting growth of tumor cells as the parent compounds. Therefore, oxidative metabolism in the liver obviously results in an enhancement of the antitumor activity and in increased solubility. Whereas in many cases metabolic oxidation results in decreased biological activity, in the present case the resulting metabolites are at least as active as the parent compounds. Thus, small variations in the substitution pattern of indirubin surprisingly gives rise to remarkable changes in the biological activity.

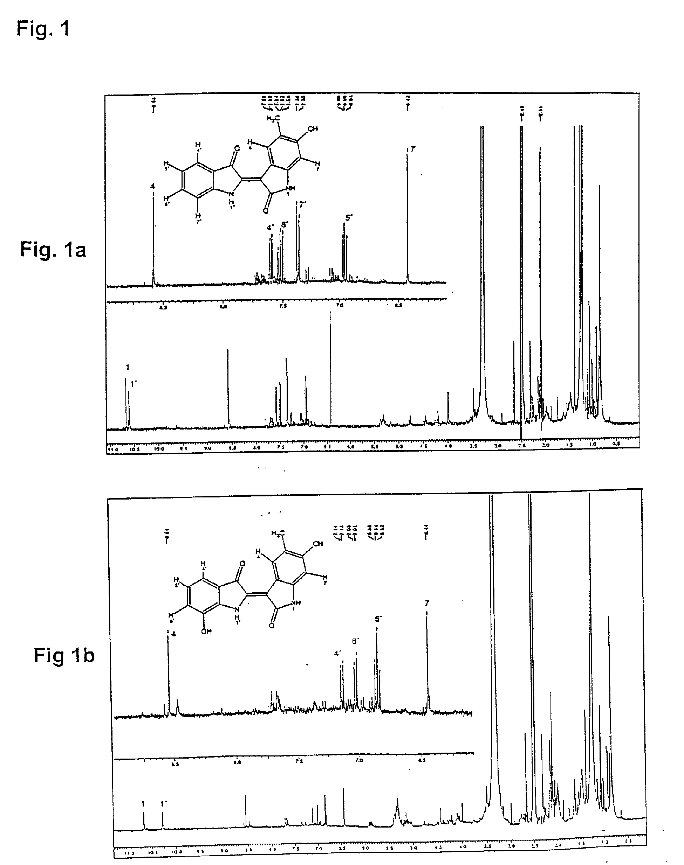

Fig. 1 shows the ¹H-NMR spectra of 6-hydroxy-5-methylindirubin (Fig. 1a) and 6,7'-dihydroxy-5-methylindirubin (Fig. 1b), each measured in DMSO-d₆.

## Claims

1. Cell membrane penetrating indirubin derivatives having the following general formula 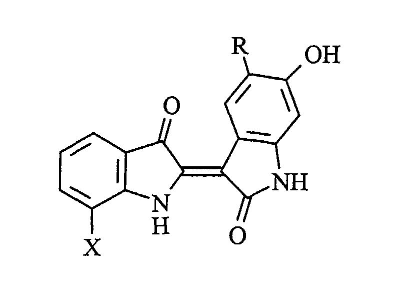 wherein the group R represents a straight-chain or branched-chain alkyl group or a straight-chain or branched-chain alkoxy group each having 1 to 18 carbon atoms, and X is H or OH.

2. Indirubin derivatives according to claim 1, wherein the group R represents a straight-chain or branched-chain alkyl group or a straight-chain or branched-chain alkoxy group each having 1 to 4 carbon atoms.

3. Indirubin derivatives according to claim 1 or 2, which are 6-hydroxy-5-methylindirubin and 6,7'-dihydroxy-5-methylindirubin.

4. Indirubin derivative according to any one of the preceding claims, wherein the indirubin derivatives is in the form of a physiologically acceptable salt.

5. Pharmaceutical formulation comprising at least one of the indirubin derivatives according to any one of claims 1 to 4.

6. Use of the pharmaceutical formulation according to claim 5 for the manufacture of a medicament for the treatment of human solid tumors and leukemia and metastases thereof.
